# EUROPEAN PATENT SPECIFICATION

(11) **EP 4 198 179 B1**
(45) Date of publication and mention of the grant of the patent: **21.08.2024**
(21) Application number: 22209761.0
(22) Date of filing: 26.11.2022
(51) Int. Cl.: A61F 13/01, D01D 5/00, D04H 1/728, A61F 13/00

(54) **A METHOD OF PRODUCING A SHRINKABLE STARCH MEMBRANE AND USE OF THE SHRINKABLE STARCH MEMBRANE IN MEDICINE AS A DRESSING**
VERFAHREN ZUR HERSTELLUNG EINER SCHRUMPFBAREN STÄRKEMEMBRAN UND VERWENDUNG DER SCHRUMPFBAREN STÄRKEMEMBRAN IN DER MEDIZIN ALS WUNDVERBAND
PROCÉDÉ DE PRODUCTION D'UNE MEMBRANE RÉTRÉCISSABLE À BASE D'AMIDON ET UTILISATION DE LA MEMBRANE RÉTRÉCISSABLE À BASE D'AMIDON EN MÉDECINE COMME PANSEMENT

(30) Priority: 30.11.2021 PL 43968321
(43) Date of publication of application: 21.06.2023
(73) Proprietor: Akademia Gorniczo-Hutnicza im. Stanislawa Staszica w Krakowie, 30-059 Krakow (PL)
(72) Inventor: STACHEWICZ, Urszula, 30-091 Kraków (PL); SROCZYK, Ewa, 38-300 Gorlice (PL)
(74) Representative: Augustyniak, Magdalena Anna

(56) References cited:
- CN-A- 113 005 641
- CN-A- 113 026 210
- US-A1- 2018 044 818
- ANICA LANCUšKI ET AL: "Rheological Properties and Electrospinnability of High-Amylose Starch in Formic Acid", BIOMACROMOLECULES, vol. 16, no. 8, 1 January 2015 (2015-01-01), US, pages 2529 - 2536, XP055477426, ISSN: 1525-7797, DOI: 10.1021/acs.biomac.5b00817
- FONSECA LAURA MARTINS ET AL: "Electrospinning of native and anionic corn starch fibers with different amylose contents", FOOD RESEARCH INTERNATIONAL, ELSEVIER, AMSTERDAM, NL, vol. 116, 11 October 2018 (2018-10-11), pages 1318 - 1326, XP085593771, ISSN: 0963-9969, DOI: 10.1016/J.FOODRES.2018.10.021

## Description

The subject matter of the invention relates to a method of producing a shrinkable starch membrane with high porosity. The subject matter of the invention also relates to the use of the shrinkable starch membrane in medicine as a dressing, for the treatment of moist, extensive and difficult-to-heal wounds.

Starch is one of the most widespread biopolymers found in nature. It is classified as a polysaccharide of plant origin and consists of glucose mers linked by α-glycosidic bonds and acts as an energy store in plants. It is used primarily as a thickening agent in the food industry. It is also used in the pharmaceutical, cosmetic and paper industries. Starch is a biodegradable polymer. When added to other polymers, it makes plastics with an addition of starch biodegradable in a very short time. Starch can be modified by physical, chemical or biochemical processes to improve its performance properties.

A biodegradable nonwoven starch-containing fabric for sanitary and food packaging materials with a controlled rate of biodegradation is known from patent description KR100824719 B1. The nonwoven fabric was produced in the process of electrospinning from a solution prepared by dissolving starch, polyvinyl alcohol and a crosslinking agent in the form of boronic acid in water.

The electrospinning method involves pulling out fibres in an electric field from a polymer solution. The solution is pushed out through a nozzle, with the flow of solution controlled by an infusion pump. A high voltage is applied to the nozzle. The potential difference between the spinning nozzle and the collector results in the solution being drawn into very thin fibres, which are collected in the form of a membrane on the collector.

A nonwoven fabric produced by the electrospinning method from 60-80 parts by weight of corn starch and 20-40 parts by weight of guar gum is known from patent application CN106436021 A. First, the ingredients were prepared. Distilled water was added to the corn starch, everything was mixed and heated at a stirring speed of 200 rpm to obtain a starch sol. Then, guar gum was added to the distilled water, the suspension was mixed and the precipitated protein impurities were centrifuged. The prepared ingredients were mixed, and the resulting solution was subjected to electrospinning. A nonwoven fabric was obtained, which can be used in the packaging for the storage of food to ensure its freshness and, additionally, it can serve as a carrier and transfer system for functional ingredients, such as a natural antimicrobial agent and antioxidant.

There is known from a publication by W. Cárdenas et al, titled: "Preparation of potato starch microfibers obtained by electro wet spinning," IOP Conf. Ser. Mater. Sci. Eng. 138: 12001, DOI: 10.1088/1757-899X/138/1/012001 a method of producing porous membranes from potato starch by the method of electrospinning into a coagulation solution. A solution of starch in dimethylsulfoxide was prepared, and the coagulation solution was an aqueous 70% ethanol solution. The coagulation solution was designed to solidify the fibres. Electrospinning into the coagulation solution was carried out using different sets of parameters (voltage, flow rate, distance between the needle and the collector). All the processes resulted in the production of connected, fused fibres with a heterogeneous morphology and diameter, and even with a lack of fibre continuity. Contact of the starch solution stream with the ethanol solution at the moment of deposition on the collector did not result in complete solidification, which was the cause of the connections between the fibres.

A method of producing and composition of starch fibres or particles in the electrospinning or electrospraying (electrospray) process into a coagulation solution is known from the publication of international application WO2013130586 A1. The composition is intended for use in drug delivery, filtration or electronics. The method comprises producing a starch solution of 1 - 40% by weight, heating it to a temperature higher than the melting point or dissolution temperature of the starch in the solvent, and then electrospinning it into the coagulation solution in order to produce and compose starch fibres or particles. The final step is washing the composition in order to remove the solvent. Preferably, the starch is dissolved in a solvent such as: DMSO, an aqueous solution of DMSO, an aqueous solution of N-methylmorpholine N-oxide (NMMO), N, N-dimethylacetamide with 3% LiCl, dimethylformamide (DMF) and an aqueous solution of DMF. The coagulation solution is preferably in the form of e.g. methanol, ethanol, 1-propanol, isopropyl alcohol, butyl alcohol, amyl alcohol, pentanol, hexanol, heptanol, or a mixture thereof. The method also comprises the addition of fillers such as, for example, drugs, pharmaceutical compositions, flavouring agents, dyes, agricultural agents, pesticides, catalysts, fluorescent dyes or combinations thereof to the coagulation solution.

A fibrous membrane for tissue regeneration, produced by the electrospinning method, formed by interweaving fibres with diameters of 10 nm-100 µm, which has a porous structure, is known from patent description EP2921136 B1. The fibres can be made of biodegradable materials, non-biodegradable materials or combinations thereof, such as polylactic acid, polycaprolactone, polyglycolic acid, polyurethane, polymethyl methacrylate, polyvinyl alcohol, starch, cellulose, alginate, among others. The method of producing a fibrous membrane comprises the following steps: dissolving a polymer in a solvent to obtain a homogeneous solution, placing the solution in a syringe and carrying out electrospinning to obtain a fibrous membrane, then subjecting it to stretching and optionally freezing and subjecting it to vacuum lyophilisation. Electrospinning is carried out at a potential difference between the needle and the collector of 5-45 kV, a distance between the needle and the collector of 5-30 cm, and a polymer solution flow rate of 0.1-15.0 ml/h.

US 2018/044818 A1 discloses a method of making a starch-formate fiber, the method comprising the steps of: providing a first spinning dope comprising a solution or dispersion of starch in a solvent comprising at least 50% vol. formic acid, where the starch is present at a concentration above the critical entanglement concentration where starch fibers are to be produced; electrospinning the spinning dope to produce a starch-formate fiber.

Unexpectedly, it has turned out that it is possible to produce a nonwoven fabric from starch that shrinks under the influence of water, which opens up completely new possibilities for its application.

The aim of the invention is to produce a shrinkable starch membrane by a simple, one-step and inexpensive method. The aim of the invention is also to use the shrinkable starch membrane in medicine as a dressing, for the treatment of moist, extensive and hard-to-heal wounds.

The gist of the method of producing a shrinkable starch membrane by the electrospinning method, from a solution that is a mixture of starch and formic acid, is characterized in that a solution having a concentration of 18-22% by weight is prepared from corn starch and concentrated formic acid, the concentration of which is 98-99% by weight, and is mixed at a temperature of 20-25 °C at a speed of 100-200 rpm for 0.5-10.0 hours. Then the homogeneous solution is allowed to rest for 15-30 hours, after which it is subjected to electrospinning in the conditions of 50-70% relative humidity, with a potential difference between the needle and the collector of 15-17 kV, a distance between the needle and the collector of 8-12 cm and a polymer solution flow rate of 0.50-0.70 ml/h, thus obtaining a membrane with a porosity of at least 60% and an average fibre diameter of 0.43-1.60 µm, which is stored at a temperature of 20-25 °C in a sealed container impermeable to humidity. The gist of the solution also relates to the use of the shrinkable starch membrane, produced by the method described in claim 1, in medicine as a dressing for the treatment of moist, extensive and difficult-to-heal wounds, after cutting out an element from it having dimensions adjusted to the wound, which is sterilized with UV radiation and optionally an agent supporting the treatment is added, and then the element is applied directly to the moist wound. The treatment substances may be disinfectants, antibiotics, ointments, creams or natural oils to support the therapy, depending on the nature of the wound.

Preferably, after application to the wound, the membrane is wetted with water having a temperature of 20-40 °C, in an amount of at least 6 µl/cm³.

The method according to the invention makes it possible to obtain membranes with unique properties, in a simple way. It is a one-step electrospinning process, for which an inexpensive, biodegradable natural polymer is used. Once made, the membrane requires no additional chemical or physical modifications. The concentration of starch and the choice of a solvent in the form of concentrated formic acid ensure that uniform fibres with diameters in the range of 0.43-1.60 µm are obtained. This is a key feature, since the diameter of the fibres directly determines the pore size of the membrane, which is important for biomedical applications. The step of setting aside the polymer solution before electrospinning for 15-30 hours allows the optimum viscosity of the solution to be obtained, which is a factor that also affects the size of the fibres and pores of the membrane. On the other hand, the ambient humidity during the membrane production process, during electrospinning, the voltage value between the needle and the collector, and the polymer flow rate, control the fibre morphology and its behaviour toward water. The membrane shrinks when exposed to moisture and then it maintains its shape permanently.

The membrane can be used in medicine as a dressing, for treating moist, extensive and hard-to-heal wounds. Starch membranes protect the wound mechanically by forming a barrier against the penetration of pathogens. In addition, they provide a reservoir of therapeutic substances, which results in their prolonged release. Since extensive wounds are usually moist, the membrane shrinks when exposed to wound moisture and skin temperature of 32 °C and simultaneously dries the wound. Such conditions are sufficient for the shrinking membrane to promote the approximation of wound edges and, consequently, wound closure, accelerating the healing process. As the membrane shrinks, its pores shrink, resulting in the extrusion of therapeutic substances from the membrane, which are absorbed by the skin. The initial porosity of the membrane is preferable, which is significant for the storage of therapeutic substances and for gas exchange between the skin and the environment. The starch membrane is a breathable, air-permeable material. Even after the membrane has shrunk, it still retains its porosity, which still enables free gas exchange. The remainder of the therapeutic substances, not extruded by the shrinkage of the membrane, enters the skin through the mechanisms of diffusion and inertia. The membrane can also be used for dry wounds. Then the membrane should be sprayed, after application, with sterile water having a temperature in the range of 20-40 °C in order to induce its shrinkage.

The method of producing the shrinkable starch membrane is explained in detail in the following examples and in the drawing, wherein Fig. 1a shows a microscopic photograph of a dry membrane produced by the method described in Example 1, Fig. 1b shows the distribution of pore diameters of this membrane, Fig. 2a shows a microscopic photograph of a starch membrane after shrinkage, Fig. 2b shows the distribution of its pore diameters, Fig. 3a shows a photograph of the dressing before shrinkage, and Fig. 3b shows a photograph of the dressing after shrinkage.

### Example 1

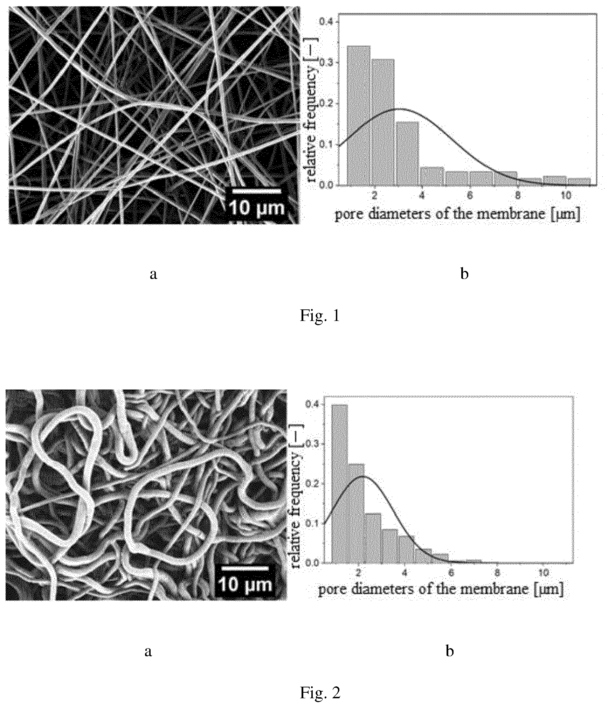

A 20% solution of corn starch in 99% formic acid was prepared. The ingredients were mixed on a magnetic stirrer at the temperature of 22 °C, at the speed of 200 rpm, until a homogeneous solution was obtained. Then, the solution was allowed to rest for 20 hours, after which time 2 ml of the solution was drawn into a syringe and the syringe was plugged with a sterile needle. A tube was connected to the needle and a second needle was connected thereto so that the tubing ended with the blunt end of the needle. A potential difference of 16 kV was created between the needle and the collector by applying a positive voltage of +14 kV to the nozzle and a negative voltage of -2 kV to the collector. The needle was positioned at a distance of 10 cm from the collector. Electrospinning was carried out for 1.5 hours under 60% relative humidity conditions at 0.60 ml/h rate of flow of the solution through the syringe. A membrane having a thickness of 25.88 ± 2.62 µm, a porosity of 73.7 ± 7.9%, an average fibre diameter of 0.73 ± 0.21 µm and a pore diameter of 3.03 ± 2.19 µm was obtained, a microscopic photo of which, together with the pore diameter distribution is presented in Fig. 1a and Fig. 1b. The produced membrane was placed in a sealed container impermeable to humidity and was stored at room temperature of 25 °C.

### Example 2

From the membrane produced by the method described in Example 1, after removing it from the packaging, a square of 4x4 cm was cut out, sterilized using UV radiation for 10 minutes and an antibiotic ointment was applied to it, and then the membrane was applied to the wound. Under the influence of the wound moisture and human body temperature, the membrane shrank and caused the wound edges to come closer together, supporting and enabling a more effective treatment thereof.

### Example 3

From the membrane produced by the method described in Example 1, after removing it from the packaging, a 4x4 cm square was cut out, sterilized using UV radiation for 10 minutes and an antibiotic ointment was applied to it, and then the membrane was applied to the wound. The dressing was wetted with distilled water (Class I) having a temperature of 20-40 °C in an amount of 96 µl. Under the influence of the wetting and skin temperature, the membrane shrank and caused the wound edges to come closer together, supporting and enabling more effective wound healing. Additionally, an agent supporting the treatment was squeezed out of the pores of the membrane as it was shrinking.

Fig. 2a and 2b show the membrane after shrinkage. Its porosity is 29.7 ± 2.9% and its pore diameter is 2.17 ± 1.33 µm.

### Example 4

From the membrane produced by the method described in Example 1, after removing it from the packaging, a 4x4 cm square was cut out, and was sterilized using UV radiation for 10 minutes. The extensive wound was sprayed with disinfectant, and then the membrane was applied to it. Under the influence wound moisture and the disinfectant, as well as human body temperature, the membrane shrank and caused the wound edges to come closer together, supporting a more effective wound treatment. Additionally, the membrane provided protection for the wound against pathogens that delay wound healing and impair the patient's health.

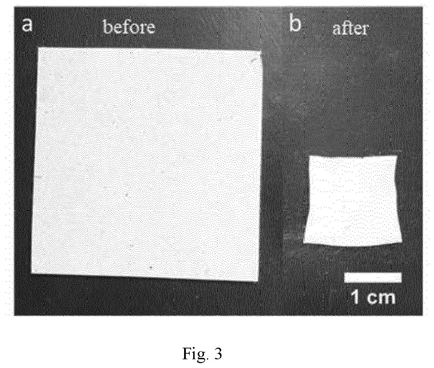

Fig. 3a shows a photograph of the dressing before shrinking, and Fig. 3b after its shrinkage.

## Claims

1. A method of producing a shrinkable starch membrane, by the electrospinning process, from a solution which is a mixture of starch and formic acid, **characterized in that** a solution having a concentration of 18-22% by weight is prepared from corn starch and concentrated formic acid, the concentration of which is 98-99% by weight, is mixed at a temperature of 20-25 °C at a speed of 100-200 rpm. for 0.5-10.0 hours, then the homogeneous solution is allowed to rest for 15-30 hours, after which it is subjected to electrospinning in the conditions of 50-70% relative humidity, with a potential difference between the needle and the collector of 15-17 kV, a distance between the needle and the collector of 8-12 cm, and a polymer solution flow rate of 0.50-0.70 ml/h, thus obtaining a membrane with a porosity of at least 60% and an average fibre diameter of 0.43-1.60 µm, which is stored at a temperature of 20-25 °C in a sealed container impermeable to humidity.

2. Use of the shrinkable starch membrane produced by the method described in claim 1 in medicine as a dressing for the treatment of moist, extensive and difficult-to-heal wounds, after cutting out an element from it having dimensions adjusted to the wound, which is sterilized with UV radiation and optionally an agent supporting the treatment is added, and then the element is applied directly to the moist wound.

3. Use according to claim 2, **characterized in that**, after application to the wound, the membrane is wetted with water having a temperature of 20-40 °C, in an amount of at least 6 µl/cm³.

## Patentansprüche

1. Verfahren zur Herstellung einer schrumpfbaren Stärkemembran durch das Elektrospinnverfahren aus einer Lösung, die eine Mischung aus Stärke und Ameisensäure ist, **dadurch gekennzeichnet, dass** eine Lösung aus Maisstärke und konzentrierter Ameisensäure, deren Konzentration 98-99 Gew.-% beträgt, mit einer Konzentration von 18-22 Gew.-%, hergestellt wird, die Lösung bei einer Temperatur von 20-25°C und einer Geschwindigkeit von 100-200 U/min 0,5-10,0 Stunden lang gemischt wird, dann man die homogene Lösung 15-30 Stunden ruhen stehen lässt, danach es dem Elektrospinnen bei 50-70% relativer Luftfeuchtigkeit, mit einer Potentialdifferenz zwischen Nadel und Kollektor von 15-17 kV und einem Abstand zwischen Nadel und Kollektor von 8-12 cm und einer Fließgeschwindigkeit der Polymerlösung von 0,50-0,70 mL/h unterzogen wird, wodurch eine Membran mit einer Porosität von mindestens 60% und einem durchschnittlichen Faserdurchmesser von 0,43-1,60 µm erhalten wird, die bei einer Temperatur von 20-25°C in einem verschlossenen, feuchtigkeitsundurchlässigen Behälter gelagert wird.

2. Verwendung der nach dem in Anspruch 1 beschriebenen Verfahren hergestellten schrumpfbaren Stärkemembran in der Medizin als Verband zur Behandlung feuchter, ausgedehnter und schwer heilender Wunden; nach dem Ausschneiden eines an die Wunde angepassten Elements wird dieses mit Ultraviolettstrahlung sterilisiert und gegebenenfalls mit einem die Behandlung unterstützenden Mittel versetzt, und anschließend wird das Element direkt auf die feuchte Wunde aufgetragen.

3. Verwendung nach Anspruch 2, **dadurch gekennzeichnet, dass** die Membran nach dem Aufbringen auf die Wunde mit Wasser mit einer Temperatur von 20-40°C in einer Menge von mindestens 6 µL/cm³ benetzt wird.

## Revendications

1. Procédé de production d'une membrane d'amidon rétractable, par le procédé d'électrofilage, à partir d'une solution qui est un mélange d'amidon et d'acide formique, **caractérisé en ce qu'**on prépare une solution ayant une concentration de 18 à 22 % en poids à partir d'amidon de maïs et de l'acide formique concentré, dont la concentration est de 98 à 99% en poids, la solution est mélangé pendant 0,5 à 10,0 heures à une température de 20 à 25°C à une vitesse de 100 à 200 tr/min, puis la solution homogène est laissée à repos pendant 15-30 heures, après quoi il est soumis à un électrofilage dans des conditions de : 50-70% d'humidité relative, avec une différence de potentiel entre l'aiguille et le collecteur de 15-17 kV, une distance entre l'aiguille et le collecteur de 8 à 12 cm et un débit de solution de polymère de 0,50 à 0,70 mL/h, obtenant ainsi une membrane avec une porosité d'au moins 60% et un diamètre moyen de fibre de 0,43 à 1,60 µm, qui est stockée à une température de 20-25°C dans un récipient fermé et imperméable à l'humidité.

2. Utilisation de la membrane d'amidon rétractable produite par le procédé décrit dans la revendication 1 en médecine comme pansement pour le traitement de plaies humides, étendues et difficiles à cicatriser ; après avoir découpé un élément ayant des dimensions adaptées à la plaie, on le stérilise par rayonnement ultraviolet et on ajoute éventuellement un agent supportant le traitement, puis on applique l'élément directement sur la plaie humide.

3. Utilisation selon la revendication 2 **caractérisée en ce que**, après application sur la plaie, la membrane est soudée avec de l'eau ayant une température de 20 à 40°C, en quantité au moins 6 µL/cm³.
